# EUROPEAN PATENT APPLICATION

(11) **EP 2 289 591 A2**
(43) Date of publication of application: **02.03.2011**
(21) Application number: 10015160.4
(22) Date of filing: 12.06.2006
(51) Int. Cl.: A61M 25/01

(54) **Endoscope treatment instrument and treatment instrument apparatus for endoscope**

(30) Priority: 14.06.2005 JP 2005174058
(62) Divisional of application: 06757256.0
(71) Applicant: Olympus Medical Systems Corporation, Shibuya-ku Tokyo 151-0072 (JP)
(72) Inventor: Ishiguro, Tsutomu, Tokyo 151-0072 (JP)
(74) Representative: Gunzelmann, Rainer

(57) **Abstract**

An endoscopic treatment instrument (11) includes: a long tube body (12) to be inserted into a body cavity, including a flexible member; a first bending portion (14) provided to a distal end part of the tube body, for bending the tube body with respect to an axial direction; a second bending portion (15) provided in a linked manner to a proximal end side of the first bending portion, for bending the tube body in the axial direction; and a bending operation portion for independently bending the first and second bending portions.

## Description

### Field of the Invention

The present invention relates to an endoscopic treatment instrument to be inserted into a lumen in a body cavity through an endoscope, allowing for insertion operation adapted to running shape of the lumen, and to a treatment instrument apparatus for endoscope including the endoscopic treatment instrument.

### Background Art

Conventionally, intra-body-cavity observations have been performed by inserting an endoscope into a body cavity, or various treatments by inserting various treatment instruments into the body cavity through a treatment instrument insertion duct provided in the endoscope.

For example, in performing a treatment checking the complicated running shapes of bile duct and pancreatic duct with contrast medium agent administered into the bile duct and pancreatic duct from duodenum papilla, the treatment instrument is protruded from a side surface at a distal end portion of an endoscope insertion portion inserted into the duodenum. At this time, an operator needs to perform insertion operation of the treatment instrument by orienting the treatment instrument in a direction to look up at the papilla through an observation window provided to the endoscope insertion portion.

To this end, at the distal end portion of the endoscope insertion portion, a raising table is provided to change the lead-out direction of the treatment instrument, i.e., to lead out the treatment instrument in the papilla direction. By adjusting raising angle of the raising table, the operator inserts the treatment instrument from the papilla into the bile duct or pancreatic duct.

Japanese unexamined patent publication No. 6-63004 shows a medical tube provided with a bending portion at a distal end of a treatment instrument, for performing insertion operation along running shapes of the bile duct and the pancreatic duct. This medical tube is configured by a first multihole tube which is flexible and forms a bending portion on a distal end side, a second multihole tube which has high hardness and joined to a rear end of the first multihole tube, and two shape-memory alloy wires arranged to face to each other along axial direction of the first multihole tube. According to this configuration, the shape-memory alloy wires are heated or cooled down to shrink or extend in length to bend the first multihole tube in two directions.

In other words, in the medical tube, shrinking and extending characteristics of the shape-memory alloy wire are used to bend the first multihole tube in two directions, so as to insert the medical tube along the shape of the bile duct and the pancreatic duct.

However, in the medical tube proposed in Japanese unexamined patent publication No. 6-63004, it is necessary that the shape-memory alloy wires, after being heated to deform into a certain shape, is cooled down to quickly return to original shape. Thus, the medical tube needs a mechanism to cool down the shape-memory alloy wires to increase outer diameter of the medical tube.

That is, it is desired to decrease the outer diameter of a treatment instrument to be inserted along the complicated running shape of the bile duct and the pancreatic duct.

The present invention has been made in view of the above circumstances, and an object of the present invention is to provide an endoscopic treatment instrument which has a small outer diameter and can facilitate insertion operation into a lumen with complicated running shape, and a treatment instrument apparatus for endoscope.

### Disclosure of Invention

### Means for Solving the Problem

An endoscopic treatment instrument of the present invention includes: a long tube body to be inserted into a body cavity, including a flexible member; a first bending portion provided to a distal end part of the tube body, for bending the tube body with respect to an axial direction; a second bending portion provided in a linked manner to a proximal end side of the first bending portion, for bending the tube body in the axial direction; and a bending operation portion for independently bending the first and second bending portions.

### Brief Description of the Drawings

FIG. 1 is a view illustrating a configuration of a treatment instrument apparatus for endoscope configured by an endoscopic treatment instrument and an endoscope.
FIG. 2 is a partial sectional view showing a configuration of a distal end portion of the endoscopic treatment instrument.
FIG. 3 is a sectional view of A-A line of FIG. 2.
FIG. 4 is a sectional view of B-B line of FIG. 2.
FIG. 5 is a front view showing a configuration of a distal end surface of the endoscopic treatment instrument of FIG. 2 as viewed from an arrow C direction.
FIG. 6 is a flow chart illustrating an exemplary control action during insertion operation of the treatment instrument apparatus for endoscope.
FIG. 7 is a sectional view illustrating arrangement positions of artificial muscles having strain sensors of a first bending portion, in a configuration in which artificial muscles having strain sensors are arranged with 45-degree phase between the first bending portion and a second bending portion.
FIG. 8 is a sectional view illustrating arrangement positions of artificial muscles having strain sensors of the second bending portion, in a configuration in which artificial muscles having strain sensors are arranged with 45-degree phase between the first and second bending portions.

### Best Mode for Carrying Out the Invention

Referring to the drawings, an embodiment of the present invention is described in detail below.

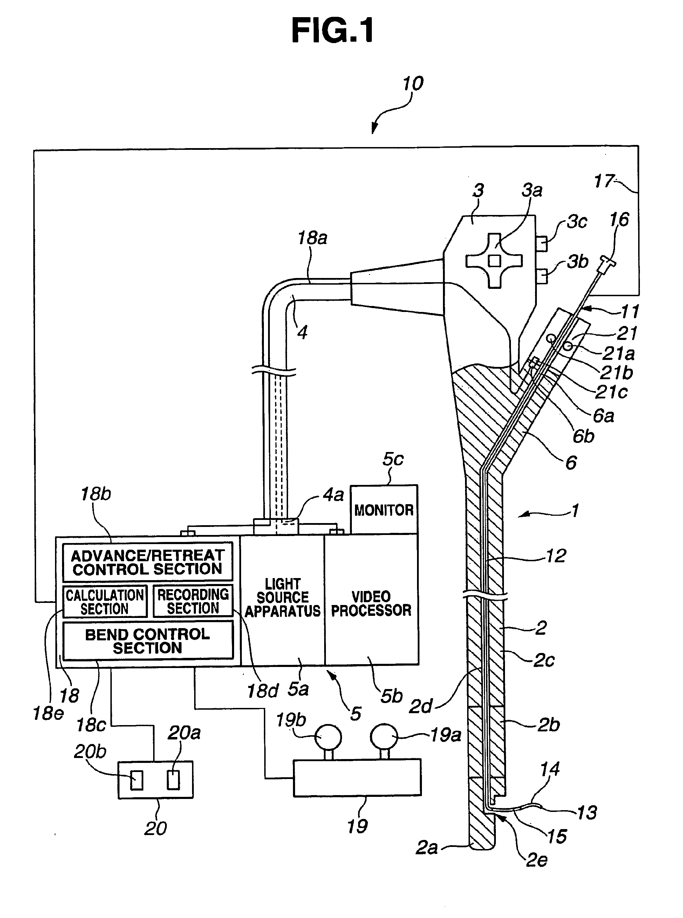

Using FIG. 1, a treatment instrument apparatus for endoscope 10 of the embodiment of the present invention is described. The treatment instrument apparatus for endoscope 10 includes an endoscopic treatment instrument 11, which is inserted into a target region in a body cavity through an endoscope 1.

The endoscope 1 includes an insertion portion 2 to be inserted in the body cavity, an operation portion 3 provided on a proximal end side of the insertion portion, and a universal cord 4 extended from the operation portion 3. An end portion of the universal cord 4 is connected with an external instrument 5. The insertion portion 2 includes, in the following order from a distal end side thereof, a distal end portion 2a, a bending portion 2b which bends in, for example, up/down and left/right directions, and a long flexible tube portion 2c formed of a flexible member. The operation portion 3, which serves also as a grasping portion to be grasped by an operator, is provided with a bending knob 3a to be operated to bend the bending portion 2b, and an air/water feeding button 3b and a suction button 3c for feeding air/water and suction from the distal end portion 2a. On the insertion portion 2 side of the operation portion 3, a treatment instrument insertion portion 6 is provided.

Though not shown, the distal end portion 2a of insertion portion 2 is provided with an image pickup optical system including a CCD as an image pickup device, an optical lens, and the like; an illumination optical system to irradiate illumination light; a nozzle to clean a surface of the optical lens such as of the image pickup optical system; a forward water feeding hole; a treatment instrument protruding hole; and a treatment instrument raising table. Note that the endoscope 1 shown in FIG. 1 is of a side-view type, wherein the image pickup optical system, the illumination optical system, and also the treatment instrument protruding hole are provided on a side surface of the distal end portion 2a. The treatment instrument raising table is configured to change lead-out direction of the treatment instrument externally protruded from the treatment instrument protruding hole of the distal end portion 2a, from lateral to upward direction in the drawing.

Though not shown, in the flexible tube portion 2c of the insertion portion 2, there are inserted a signal cable to transmit a signal to drive the CCD of the image pickup optical system and an image pickup signal; a light guide cable to lead illumination light to the illumination optical system; a bending wire joined to the bending portion 2b and the bending knob 3a; various tubes for feeding water/air and suction; and others, and is provided a treatment instrument insertion duct 2d to communicate between a treatment instrument insertion port 6a and a treatment instrument protruding hole 2e provided at the distal end portion 2a.

In through the universal cord 4 are inserted the signal cable, the light guide cable, the various tubes, signal lines extended from the air/water feeding button and the suction button, and so on. The end portion of the universal cord 4 is provided with a connector 4a which is detachably attachable to the external instrument 5.

The external instrument 5 connected with the connector 4a includes a light source apparatus 5a and a video processor 5b. The light source apparatus 5a generates illumination light to be supplied to the illumination optical system. The video processor 5b supplies a drive signal of the CCD, and generates and records video signals based on image pickup signals from the CCD. The video processor 5b is connected to a monitor 5c as the external instrument 5. On a screen of the monitor 5c, an endoscope image or the like is displayed based on the video signal generated at the video processor 5b. Note that the light source apparatus 5a incorporates or is attached with an air/water feeding pump and a suction pump.

By connecting the universal connector 4a of the universal cord 4 to each of the instruments 5a, 5b, the signal lines and the tubes are connected in a predetermined function state.

The operator inserts the insertion portion 2 of the endoscope 1 into the body cavity, observing an endoscope image displayed on the monitor 5c. After the distal end portion 2a of the insertion portion 2 is inserted to near the target region in the body cavity, the endoscopic treatment instrument 11 is inserted into the treatment instrument insertion port 6a, through the treatment instrument insertion duct 2d, to protrude from the treatment instrument protruding hole 2e of the distal end portion 2a.

Here, the endoscopic treatment instrument 11 of the present invention is described.

The endoscopic treatment instrument 11 includes a tube body 12, pressure sensors 13, a first bending portion 14, a second bending portion 15, an end cap 16, a pressure sensor signal cable 17, and a control unit 18. The tube body 12 is a long and hollow tube having a through hole 12a. The through hole 12 serves as an insertion path in which are inserted a guide wire (not shown) or a treatment instrument such as a catheter, or as a flow path to supply a fluid such as an X-ray contrast medium. The pressure sensors 13 serve as pressure measuring means, and for example four pressure sensors 13 are provided at the distal end part, specifically on a distal end surface, of the tube body 12. The first bending portion 14 is provided by a predetermined length in axial direction at the distal end part of the tube body 12. The second bending portion 15 is provided by a predetermined length in axial direction on a rear end side of the first bending portion 14. The end cap 16 is provided at a proximal end of the tube body 12. The signal cable 17 is formed to bundle signal lines (not shown) extended from the pressure sensors 13, the first bending portion 14, and the second bending portion 15. An end portion of the signal cable 17 is connected to the control unit 18.

The control unit 18 is control means and connected with a joystick apparatus 19 and a foot switch 20. The joystick apparatus 19 is bending operation means for independent bending operations of the first bending portion 14 and the second bending portion 15. The foot switch 20 is advancing/retreating means to activate an advancing/retreating apparatus 21 for advancing/retreating the tube body 12 of the endoscopic treatment instrument 11.

The advancing/retreating apparatus 21 is provided to the treatment instrument insertion portion 6 of the operation portion 3 of the endoscope apparatus 1. The advancing/retreating apparatus 21 includes a pair of roller 21a, 21b to sandwichingly hold the tube body 12. One of the rollers 21a, 21b is forwardly/backwardly rotated by an electric motor not shown. By rotating the electric motor with the tube body 12 sandwichingly held by the pair of rollers 21a, 21b, the tube body 12 is moved to advance/retreat. The advancing/retreating apparatus 21 includes an electrode portion 21c to electrically contact a contact portion 6b provided to the treatment instrument insertion portion 6. From the contact portion 6b extends a signal cable 18a. The signal cable 18a includes a power line to supply electric power to the electric motor, and a signal line to transmit a control signal to instruct a rotation action. The signal cable 18a is inserted in through the operation portion 3 and the universal cord 4, extended to the universal connector 4a, and connected to the control unit 18 via the universal connector 4a. The foot switch 20 includes switches 20a, 20b. A first switch 20a instructs an advancing action and a second switch 20b instructs a retreating action. When operated by the operator, the switches 20a, 20b outputs a signal to instruct the control unit 18 on a predetermined action. Thereafter, a control signal to rotate the roller 21a, for example, is outputted from the control unit 18 to the electric motor of the advancing/retreating apparatus 21, so that the tube body 12 is advanced/retreated.

The tube body 12 of the endoscopic treatment instrument 11 is inserted into the treatment instrument insertion duct 2d via the advancing/retreating apparatus 21 provided to the treatment instrument insertion portion 6 of the operation portion 3 of the endoscope apparatus 1. The tube body 12 is moved to advance/retreat along with operation of the foot switch 20. The first bending portion 14 and the second bending portion 15 provided at the distal end portion of the tube body 12 configuring the endoscopic treatment instrument 11, which are protruded from the treatment instrument protruding hole 2e of the distal end portion 2a, are activated to bend along with operation of joysticks 19a, 19b of the joystick apparatus 19. From the joystick apparatus 19, a signal to instruct bending action is outputted to the corresponding bending portion 14, 15 via the control unit 18.

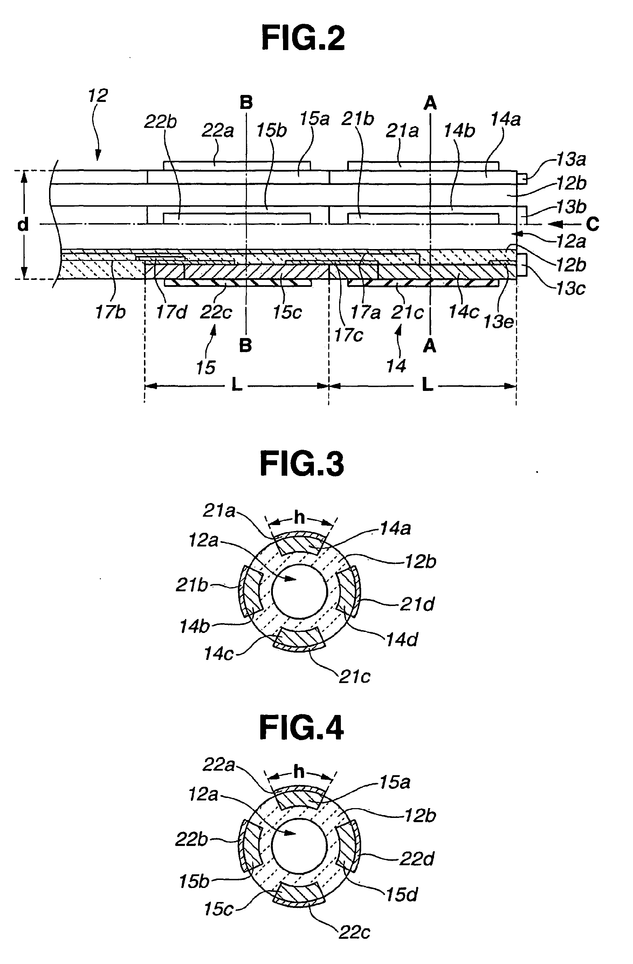

Referring to FIGS. 2 to 6, the pressure sensors 13, the first bending portion 14, and the second bending portion 15 of the endoscopic treatment instrument 11 are described.

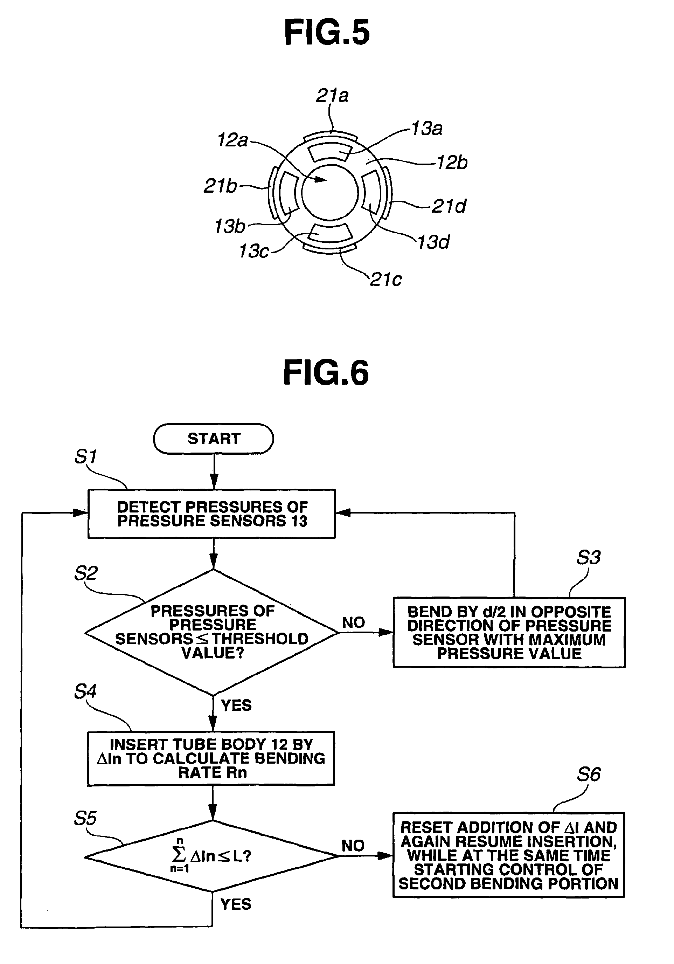

On a distal end surface of a substance portion 12b of the tube body 12, for example, four pressure sensors 13a, 13b, 13c, 13d are arranged at an interval of about 90 degrees, as shown in FIGS. 2 and 5. The pressure sensors 13a, 13b, 13c, 13d are for detecting a contact pressure to occur when the distal end surface of tube body 12 contacts a lumen wall when the tube body 12 is inserted into the lumen. That is, the pressure sensors 13a, 13b, 13c, 13d are configured to have a shape and area to detect the pressure to occur when the distal end surface of the tube body 12 contacts the lumen wall or the like.

The tube body 12 is provided with the first bending portion 14 configured with a length dimension L, and the second bending portion 15 which is provided in a linked manner to the first bending portion 14, having the same length dimension L as with the first bending portion 14. The bending portions 14, 15 are each provided with four bending mechanism portions at an interval of about 90 degrees, as shown in FIGS. 3 and 4. The bending mechanism portions are what are known as artificial muscles 14a, 14b, 14c, 14d, 15a, 15b, 15c, 15d, serving as polymeric actuators. The first bending portion 14 includes the artificial muscles 14a, 14b, 14c, 14d, and the second bending portion 15 includes the artificial muscles 15a, 15b, 15c, 15d. The polymeric actuators are formed such that, when a voltage is applied thereto, positive ions in a polymeric electrolyte move to a cathode side, resulting in swelling of the front and rear lateral portions of the actuators and therefore bending and deformation thereof. The artificial muscles 14a, 14b, 14c, 14d, 15a, 15b, 15c, 15d are formed to have a predetermined width dimension h with the length L. Note that the pressure sensors 13a, 13b, 13c, 13d, the artificial muscles 14a, 14b, 14c, 14d, and the artificial muscles 15a, 15b, 15c, 15d are arranged at similar positions as viewed from the front, in other words, in the same phase in a cross sectional direction.

On outer surfaces of the artificial muscles 14a, 14b, 14c, 14d, 15a, 15b, 15c, 15d configuring the first bending portion 14 and the second bending portion 115, there are respectively provided the strain sensors 21a, 21b, 21c, 21d, 22a, 22b, 22c, 22d having a length dimension shorter than L. The strain sensors are bending shape measuring means, which measure bending state of the bending portions 14, 15 in bent state.

To bend to deform the first bending portion 14 in, for example, upward direction in the drawing, a voltage is applied to each of the artificial muscle 14a and the artificial muscle 14c opposite to the artificial muscle 14a so as to bend the artificial muscles 14a, 14c in the same direction. That is, by adequately controlling a voltage to apply to each of the artificial muscles 14a, 14b, 14c, 14d of the first bending portion 14, the first bending portion 14 can be bent in up/down and left/right directions. Note that the second bending portion 15 can also be similarly bent in up/down and left/right directions by adequately controlling a voltage to apply to each of the artificial muscles 15a, 15b, 15c, 15d.

The strain sensors 21a, 21b, 21c, 21d detect strain that occurs with bending action of the artificial muscles 14a, 14b, 14c, 14d provided to the first bending portion 14. On the other hand, the strain sensors 22a, 22b, 22c, 22d detect strain that occurs with bending action of the artificial muscles 15a, 15b, 15c, 15d provided to the second bending portion 15. From the strain detected by these strain sensors 21a to 21d and 22a to 22d, respective curvatures of the bending portions 14, 15 are calculated by a calculation section 18a of the control unit 18 and recorded in a recording section 18d based on the calculation results.

From the end cap 16 side of the tube body 12, the signal cable 17 is extended. A proximal end of the signal cable 17 is connected to the control unit 18. In the signal cable 17, electric wires 17a, 17b and signal lines 13e, 17c, 17d are contained. The electric wire 17a is connected to each of the artificial muscles 14a, 14b, 14c, 14d configuring the first bending portion 14. The electric wire 17b is connected to each of the artificial muscles 15a, 15b, 15c, 15d configuring the second bending portion 15. These electric wires 17a, 17b supply bending action voltage to activate the bending portions 14, 15 to bend.

In contrast thereto, the signal line 13e is connected to each of the pressure sensors 13a, 13b, 13c, 13d. The signal line 13e transmits pressure detection signal detected by the pressure sensors 13a, 13b, 13c, 13d. The signal line 17c is connected to each of the strain sensors 21a, 21b, 21c, 21d, to transmit a detection value corresponding to the bending state. The signal line 17d is connected to each of the strain sensors 22a, 22b, 22c, 22d, to transmit a detection value corresponding to the bending state.

The control unit 18, the joystick apparatus 19, and the foot switch 20 are operation portions of the treatment instrument apparatus for endoscope 20. The joystick apparatus 19 includes a first joystick 19a that outputs an instruction signal to operate to bend the first bending portion 14, and a joystick 19b that outputs an instruction signal to operate to bend the second bending portion 15. Based on difference in the direction of the inclining operation and inclining angle as the operation amount of the joysticks 19a, 19b, the control unit 18 generates a bending action voltage to control bending directions and curvatures of the first bending portion 14 or the second bending portion 15. The generated bending action voltage is then applied via the electric wires 17a, 17b to the artificial muscles 14a, 14b, 14c, 14d of the first bending portion 14, or the artificial muscles 15a, 15b, 15c, 15d of the second bending portion 115, to activate the bending portions 14, 15 to bend.

The foot switch 20 outputs an instruction signal to activate the advancing/retreating apparatus 21 to rotate forward, and an instruction signal to activate the same to rotate backward. When the first switch 20a is operated to turn on, the control unit 18 outputs to the electric motor of the advancing/retreating apparatus 21 a control signal to drive the electric motor to rotate forward, via the signal cable 18a. Then, when the second switch 20a is turned off, the electric motor stops driving. On the other hand, when the second switch 20b is operated to turn on, the control unit 18 outputs to the electric motor of the advancing/retreating apparatus 21 a control signal to drive the electric motor to rotate backward, via the signal cable 18a. Then, when the first switch 20a is brought into an off state, the electric motor stops driving.

The control unit 18 includes an advance/retreat control section 18b as advance/retreat control means and a bend control section 18c as bend control means. As mentioned above, the advance/retreat control section 18b generates a control signal based on the instruction signal from the foot switch 20, while at the same time generates a control signal based on measurement results outputted from the pressure sensors 13a, 13b, 13c, 13d, so as to activate the advancing/retreating apparatus 21 to advance/retreat the tube body 12. On the other hand, the advance/retreat control section 18b generates a bending action voltage based on the instruction signal outputted from the joysticks 19a, 19b of the joystick apparatus 19, while at the same time generates a bending action voltage in line with detection values corresponding to the bending state, outputted from the strain gauges 21a to 21d, 22a to 22d, so as to activate the corresponding bending portions 14, 15 to bend in a predetermined direction.

Referring to FIG. 6, there is described an exemplary control by the advance/retreat control section 18b and the bend control section 18cb of the control unit 18.

First, the tube body 12 of the endoscopic treatment instrument 11 is inserted into the treatment instrument insertion duct 2d of the insertion portion 2 from the treatment instrument insertion port 6a of the operation portion 3 via the advancing/retreating apparatus 21 mounted at the treatment instrument insertion port 6 of the endoscope apparatus 1. Then, the distal end of the tube body 12 is protruded from the distal end portion 2a of the insertion portion 2. Thereafter, the treatment instrument raising table (not shown) provided to the distal end portion 2a is operated to raise so as to insert the tube body 12 into, for example, the bile duct via the papilla. On confirming on the screen that the distal end of the tube body 12 is inserted into the bile duct, the operator operates to turn on the first switch 20a of the foot switch 20. This results in a control signal to be outputted from the control unit 18 to the advancing/retreating apparatus 21, to drive to rotate the electric motor, advancing the tube body 12 toward a deep part of the bile duct.

As the advancing/retreating apparatus 21 is driven to start advancing the tube body 12, control by the control unit 18 starts. In other words, the control unit 18 obtains pressure values detected by the pressure sensors 13a, 13b, 13c, 13d provided on the distal end surface of the tube body 12 as shown in step S1, then proceeding to step S2. In step S2, by means of the calculation section 18a, the control unit 18 judges whether or not the pressure value obtained from each of the sensors 13a, 13b, 13c, 13d is equal to or smaller than a threshold value, that is, "pressure value of each pressure sensor ≤ threshold value", while also judging whether or not the pressure values of the pressure sensors 13a, 13b, 13c, 13d are generally the same.

Note that the threshold value here is a value with a magnitude that prevents damage to the lumen wall. Therefore, even if the pressure value has reached the threshold value, the bile duct wall or the like is not damaged by advancing of the tube body 12 in contact with the lumen wall.

In step S2, when the tube body 12 is smoothly advancing in a straight part of the bile duct, the pressure sensors 13a, 13b, 13c, 13d provide pressure values that are generally the same and smaller than the threshold value. In contrast to this, when the tube body 12 is advancing in a curving part of the bile duct, at least a part of the distal end surface of the tube body 12 contacts with the curving bile duct wall. This results in that, among the pressure sensors 13a, 13b, 13c, 13d, for example, the pressure sensor 13a in contact with the bile duct wall has a detected pressure value that is higher than detected pressure values of the other pressure sensors 13b, 13c, 13d. Thus causes the control unit 18 to judge that the side having the pressure sensor 13a on the distal end surface is in contact with the lumen wall.

The control unit 18 thus compares the pressure values detected by the pressure sensors 13a, 13b, 13c, 13d to judge whether or not the distal end surface of the tube body 12 is in contact with the bile duct wall or the like.

In the step S2, if the respective pressure values detected by the pressure sensors 13a, 13b, 13c, 13d are equal to or smaller than the threshold value, the control unit 18 judges that the distal end surface of the tube body 12 is not in contact with the bile duct wall, and proceeds to step S4. In step S4, the control unit 18 outputs, from the advance/retreat control section 18b to the advancing/retreating apparatus 21, a control signal to drive to rotate forward the advancing/retreating apparatus 21, and obtains detection values of the strain sensors 21a, 21b, 21c, 21d provided to the first bending portion 14, to calculate a curvature Rn by means of the calculation section 18a. That is, the output of the control signal to drive to rotate forward the advancing/retreating apparatus 21 makes the tube body 12 advance by Δln. For each advance by the distance Δln of the tube body 12, the control unit 18 obtains a detection value of each of the strain sensors 21a, 21b, 21c, 21d provided in the first bending portion 14, so as to calculate the curvature Rn of the first bending portion 14 from the strain amount.

Then, as shown in step S5, in step S4, the control unit 18 makes addition of Δln which is the advance amount of the tube body 12, and judges whether or not the resulting addition is equal to or smaller than the entire length L in the axial direction of the first bending portion 14. In this step S5, if the resulting addition of Δln is judged to be equal to or smaller than the entire length L of the first bending portion 14, the processing proceeds to step S1.

On the other hand, if the control unit 18 detects, in step S2, a pressure equal to or greater than the threshold value of any of the pressure sensors 13a, 13b, 13c, 13d, the control unit 18 proceeds to step S3. In step S3, the control unit 18 performs control to cause the screen to display position of, for example, the pressure sensor 13a that has detected a pressure equal to or greater than the threshold value; cause the screen to display a bending direction opposite to that of the position of the pressure sensor 13a; prompt an operation of the joystick 18a of the joystick apparatus 18; and apply a bending action voltage to the artificial muscles 14a, 14c to bend the first bending portion 14 in the direction opposite to that of the position of the pressure sensor 13a. At this time, the first bending portion 14 is to be bent by half a diameter d of the tube body 12 shown in FIG. 2.

The control unit 18 activates the first bending portion 14 to bend in the direction opposite to that of the setting position of, for example, the pressure sensor 13a that has detected a pressure greater than the threshold value in the step S3, and then returns to step S1 again.

Meanwhile, if the control unit 18 judges, in the step S5, that a resulting addition value of the advance by Δln of the tube body 12 is equal to or greater than the entire length L of the first bending portion, then proceeds to step S6. In step S6, the control unit 18 extracts from the recording section 18d data of the curvature Rn in the advancing by Δln of the first bending portion 14, thereafter continuing the advancing and insertion of the tube body 12. The bending operation of the joystick 19b of the joystick apparatus 19 is performed based on the data of the curvature Rn in the advancing by Δln of the first bending portion 14, extracted in the control unit 18. Thus, the second bending portion 15 is bend-controlled based on the data recorded in the recording section 18d by the control unit 18. This results in that the second bending portion 15 traces the path on which the first bending portion 14 advanced, which allows efficiently and smoothly performing the bending action of the second bending portion 15.

As described above, in the endoscopic treatment instrument 11 of the embodiment of the present invention, the plurality of pressure sensors 13a, 13b, 13c, 13d are provided on the distal end surface of the tube body 12 to be inserted in the lumen, and in addition, the first bending portion 14 including the artificial muscles 14a, 14b, 14c, 14d adjacently provided in the axial direction of the tube body 12, and the second bending portion 15 including the artificial muscles 15a, 15b, 15c, 15d are respectively provided with the strain sensors 21a to 21d and the strain sensors 22a to 22d for detecting the respective curvatures of the bending portions 14, 15. Therefore, it is made possible to quickly and smoothly perform the insertion operation into the complicatedly bent lumen without damaging the lumen wall by the advance of the tube body 12.

Note that, as shown in FIGS. 2 to 4, the artificial muscles 14a, 14b, 14c, 14d provided to the first bending portion 14 and the strain sensors 21a, 21b, 21c, 21d provided on the respective outer surfaces of the artificial muscles 14a, 14b, 14c, 14d, and the artificial muscles 15a, 15b, 15c, 15d provided to the second bending portion 15 and the strain sensors 22a, 22b, 22c, 22d provided on the respective outer surfaces of the strain sensors 22a, 22b, 22c, 22d, are arranged at positions in the same phase in a cross sectional direction. With such arrangement, in the case of performing a bending operation in, for example, the upward direction in the drawing, the artificial muscles 14a, 14c of the bending portion 14 and the artificial muscle 15a, 15c of the bending portions 15 are operated to be contract simultaneously. This allows the bending operation in the upward direction to be performed more quickly. Note that, also in other directions, similar working and effect can be obtained by simultaneouly operating the artificial muscles in the same phase.

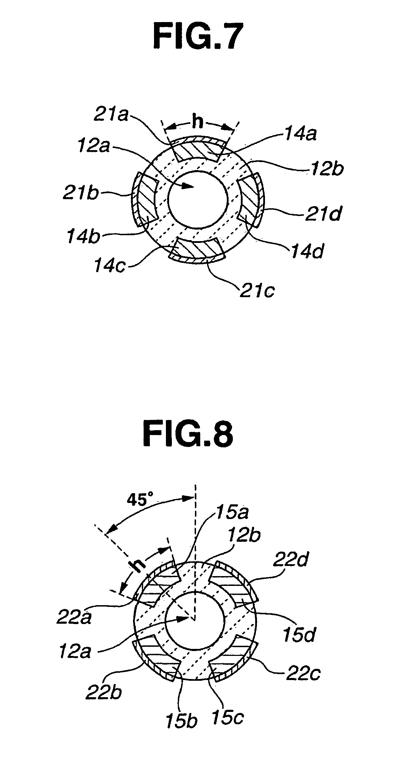

Meanwhile, the artificial muscles 14a, 14b, 14c, 14d provided to the first bending portion 14 and the strain sensors 21a, 21b, 21c, 21d provided on respective outer surfaces of the artificial muscles 14a, 14b, 14c, 14d, shown in FIG. 7, and the artificial muscle 15a, 15b, 15c, 15d provided to the second bending portion 15 and the strain sensors 22a, 22b, 22c, 22d provided on respective outer surfaces of the artificial muscle 15a, 15b, 15c, 15d, shown in FIG. 8, may be arranged in a manner shifted by, for example, 45 degrees, i.e., in a changed phase in a cross section. With such arrangement employed, when performing a bending operation in, for example, upper-right diagonal direction in FIG. 7, the artificial muscles 14a, 14d of the first bending portion 14 are simultaneously contracted, while contracting the artificial muscle 15d of the second bending portion 15. By thus controlling the three artificial muscles, bending operation in a diagonal direction can be realized.

In the case of performing a bending operation in the same diagonal direction as above using the arrangement of the artificial muscles shown in FIGS. 3 and 4, a total of four artificial muscles are controlled: the artificial muscles 14a, 14d of the first bending portion 14 and the artificial muscles 15a, 15d of the second bending portion 15. Therefore, the configuration in FIGS. 7 and 8 can better reduce the load on the control circuit provided in the control unit 18.

The above embodiment of the present invention has described an exemplary configuration in which the first bending portion 14 and the second bending portion 15 are respectively provided with four artificial muscles 14a, 14b, 14c, 14d and 15a, 15b, 15c, 15d, at an interval of 90 degrees. The interval of providing the artificial muscles, however, is not limited to 90 degrees, that is, four intervals. For example, three artificial muscle may be provided at an interval of 120 degrees, or two artificial muscles may be provided at an interval of 180 degrees. That is, the artificial muscles may be arranged in any manner to allow the distal end part of the tube body 12 to bend in up/down direction or left/right direction.

Further, the bending mechanism portions of the first bending portion 14 and the second bending portion 15 employ artificial muscles, i.e., polymeric actuators. The bending mechanism, however, is not limited to the artificial muscle, but may be one configured by a shape-memory alloy to perform a bending action, or actuators not needing cooling means such as an air pressure actuator to be activated to bend by air pressure, or a wire-driven actuator to be activated to bend by a pulling wire.

Furthermore, the pressure sensors 13a, 13b, 13c, 13d may be arranged on a tapered surface of, for example, 45 degrees provided on the end surface of the tube body 12.

Thus, it is possible to select an optimum artificial muscle arrangement for use purpose, bending frequency in any bending direction, or the like, of the tube body 12

Note that the present invention is not limited only to the above-mentioned embodiment, but may be modified and embodied in various forms without departing from the scope of the present invention.

This application is filed claiming priority from Japanese Patent Application No. 2005-174058 applied in Japan on June 14, 2005, the disclosed contents of which being incorporated in the present specification, claims, and drawings.

## Claims

1. A treatment instrument apparatus for an endoscope, comprising:
an endoscopic treatment instrument including pressure measuring means provided to a distal end part of a flexible tube body which is inserted into a body cavity through a treatment instrument insertion duct of the endoscope, the pressure measuring means measuring a pressure applied to a distal end surface of the tube body;
advancing/retreating means for performing advance/retreat actions when inserting and pulling the tube body into and out of the body cavity through the treatment instrument insertion duct; and
advance/retreat control means for controlling action state of the advancing/retreating means.

2. The treatment instrument apparatus for endoscope according to Claim 1, wherein a plurality of the pressure measuring means are provided on the distal end surface.

3. The treatment instrument apparatus for endoscope according to Claim 1, wherein the advance/retreat control means outputs a control signal for instructing the advancing/retreating means to perform any of advancing, retreating, or stopping action, based on a measurement result of the pressure measuring means.

4. The treatment instrument apparatus for endoscope according to Claim 1, wherein the advancing/retreating means is disposed to a treatment instrument insertion portion provided to an operation portion of the endoscope.

5. A treatment instrument apparatus for an endoscope, comprising:
an endoscopic treatment instrument including,
a long tube body having a flexible member, which is inserted into a body cavity through a treatment instrument insertion duct of the endoscope, the long tube body including a first bending portion at a part on a distal end side, which is bendable with respect to an axial direction, and a second bending portion provided in a linked manner on a proximal end side of the first bending portion, which is bendable with respect to the axial direction, and
pressure measuring means provided to a distal end part of the tube body, for measuring a pressure applied to a distal end surface of the tube body;
bending operation means for independently bending the first and second bending portions included by the endoscopic treatment instrument;
advancing/retreating means for performing advance/retreat actions when inserting and pulling the tube body into and out of the body cavity through the treatment instrument insertion duct; and
advance/retreat control means for controlling action state of the advancing/retreating means, based on a measurement result of the pressure measuring means.

6. A treatment instrument apparatus for an endoscope, comprising:
an endoscopic treatment instrument including,
a long tube body having a flexible member, which is inserted into a body cavity through a treatment instrument insertion duct of the endoscope, the long tube body including a first bending portion at a part on a distal end side, which is bendable with respect to an axial direction, and a second bending portion provided in a linked manner on a proximal end side of the first bending portion, which is bendable with respect to the axial direction, and
pressure measuring means provided to a distal end part of the tube body, for measuring a pressure applied to a distal end surface of the tube body;
advancing/retreating means for performing advance/retreat actions when inserting and pulling the tube body into and out of the body cavity through the treatment instrument insertion duct;
advancing/retreating means for performing advance/retreat actions when inserting and pulling the tube body into and out of the body cavity through the treatment instrument insertion duct; and
bend control means for outputting a bending action signal to the bending mechanism portions, based on a measurement result of the bending shape measuring means.

7. A treatment instrument apparatus for an endoscope, comprising:
a long tube body to be inserted into a body cavity, formed of a flexible member;
a plurality of pressure measuring means provided to a part on a distal end side of the tube body;
a first bending portion provided to a distal end part of the tube body, for bending the tube body with respect to an axial direction;
a second bending portion provided in a linked manner to a proximal end side of the first bending portion, for bending the tube body in the axial direction
a strain sensor provided to each of the first and second bending portions, for detecting respective strains of the first and second bending portions; and
control means for controlling insertion of the tube body and bending of the first or second bending portion, by detecting insertion state and bending state of the distal end part of the tube body from a pressure detected by the pressure measuring means and a strain detected by the strain sensor.

8. The treatment instrument apparatus for endoscope according to Claim 7, wherein the first and second bending portions each include a plurality of bending mechanism portions each configured of any of a polymeric actuator, a shape-memory alloy, a fluid-pressure actuator, or a wire-driven actuator.

9. The treatment instrument apparatus for endoscope according to Claim 7 or 8, wherein
the bending mechanism portions provided to the first and second bending portions are provided at equal intervals of one of 90-degree intervals, 120-degree intervals, or 180-degree intervals with respect to a circumferential direction, centering a center axis of the tube body, and
a proximal end of the first bending portion and a distal end of the second bending portion are provided in a linked manner or with a predetermined space therebetween.

10. The treatment instrument apparatus for endoscope according to Claim 7, wherein the control means comprises:
advance/retreat control means for controlling advance/retreat actions of inserting the tube body into a body cavity; and
bending operation means for controlling to independently activate and bend the first and second bending portions.

11. The treatment instrument apparatus for endoscope according to Claim 10, wherein the control means controls, along with the advance/retreat control of the tube body by the advance/retreat control means, bending action of the first bending portion or bending action of the second bending portion by means of the bending operation means, based on a pressure detected by the pressure measuring means and on a curvature of the first bending portion calculated from a strain of the first bending portion detected by the strain sensor, or a curvature of the second bending portion calculated from a strain of the second bending portion.

12. The treatment instrument apparatus for endoscope according to Claim 10 or 11, wherein, in insertion control operation of the tube body, the control means records in a recording section an advancing/retreating amount of the first bending portion based on the advance/retreat control means and a curvature of the first bending portion by bending action by the bending operation means, and operates advance or retreat of the second bending portion which is inserted subsequent to the first bending portion, based on the advancing/retreating amount and the curvature of the first bending portion recorded in the recording section.
